(19)

Europäisches Patentamt
European Patent Office
Office européen des brevets

(11) **EP 4 686 418 A1**

(12) **EUROPEAN PATENT APPLICATION**

(43) Date of publication:
**04.02.2026 Bulletin 2026/06**

(21) Application number: 25193399.0

(22) Date of filing: **01.08.2025**

(51) International Patent Classification (IPC):
***A23L 29/244*** (2016.01)   ***A23L 33/21*** (2016.01)
***A23L 5/20*** (2016.01)

(52) Cooperative Patent Classification (CPC):
**A23L 29/244; A23L 5/20; A23L 33/21**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **02.08.2024 JP 2024128105**

(71) Applicant: **Yukiguni Aguri Co., Ltd.
Numata-shi, Gunma 378-0004 (JP)**

(72) Inventors:
• **AKIMOTO, Eri
Numata-shi, Gunma, 3780126 (JP)**
• **MUROI, Fumihiro
Numata-shi, Gunma, 3780126 (JP)**

(74) Representative: **Schulz Junghans
Patentanwälte PartGmbB
Großbeerenstraße 71
10963 Berlin (DE)**

(54) **FUNCTIONAL COMPOSITION AND METHOD OF PRODUCING THE SAME**

(57)   The objective of the present invention is to provide a functional composition containing mannose and/or mannooligosaccharide and having the reduced sulfur dioxide content. The objective can be achieved to decompose konjac flour derived from konjac potatoes, which is a raw material to obtain a composition containing mannose and/or mannooligosaccharide followed by subjecting the composition to heat treatment at a temperature of 105 °C or higher to reduce the sulfur dioxide content in the functional composition. The functional composition with sulfur dioxide (sulfurous acid) reduced can be distributed as foods and beverages without any legal or regulatory problem.

EP 4 686 418 A1

**Description**

Technical Field

The present invention relates to a functional composition containing mannose and/or mannooligosaccharide, in particular a functional composition having a sulfur dioxide content of equal to or less than 0.03 g/kg.

Background Art

Konjac flour (konjac powder) is produced as a rough powder by washing, slicing, and flame drying konjac potatoes, and then as a refined powder by subjecting the rough powder to refinement treatment using a mechanical method or a pestle and mortar method. Since the drying manner is flame drying and uses heavy oil as the fuel, the sulfur content remains in the rough powder and *tobiko powder* as well as the refined powder. Therefore, the Food Sanitation Law specially stipulates that konjac flour may contain sulfur dioxide of 0.9 g/kg or less. In addition, the maximum residue level of sulfur dioxide in general foods is set at 0.03 g/kg or less (Non-Patent Document 1). When producing a konjac product from the refined powder, the refined powder is added to the product at about 3 %. If the refined powder contains 0.9 g/kg of residual sulfur dioxide, the sulfur dioxide content in the final konjac product is below the standard level of 0.03 g/kg. In this way, the residual sulfur dioxide concentration in the refined powder is regulated such that the product is safe.

While the refined powder is used in the konjac product without any problem caused, the case where the refined powder or other such powder is directly used as a konjac product causes the problem that it is difficult to place such a konjac product on the market as a food unless the sulfur dioxide content of the product is reduced to the standard level for general foods of 0.03 g/kg or less.

Mannose and mannooligosaccharide have many effects such as urinary tract infection prevention (Patent Document 1), anticancer effect (Non-Patent Document 2), and subcutaneous and visceral fat reducing effects (Patent Document 2), resulting in the attracted sugars in recent years. Several methods of producing mannose and mannooligosaccharide are disclosed. Such methods include, for example, a method of producing mannose or mannooligosaccharide using acetic acid or formic acid (Patent Document 3), and a method of producing mannose by adding hemicellulase to a refined powder of konjac potatoes (Patent Document 4). Furthermore, Patent Documents 5 and 6 disclose a method for extracting mannose and a method of producing mannan oligosaccharide, respectively. Non-Patent Document 1 dislcoses D(+)-mannose.

Citation List

Non-Patent Documents

[Non-Patent Document 1] Japan Society for Food Hygiene and Safety (ed.), Standards and Criteria for Foods and Food Additives (excerpt).
[Non-Patent Document 2] P. S. Gonzalez, et al., Mannose impairs tumour growth and enhances chemotherapy. NATURE 563, 719-723 (2018).
[Non-Patent Document 3] D(+)-mannose; internet; URL<https://labchem-wako.fujifilm.com/jp/product/detail/W01W0113-0087.html>

Patent Documents

[Patent Document 1] JP 2011-219469 A
[Patent Document 2] JP 4488852 B
[Patent Document 3] JP 5960381 B
[Patent Document 4] JP 2011-254753 A
[Patent Document 5] JP 2019-199462 A
[Patent Document 6] JP 2004-254646 A

Summary of the Invention

Problems to be Solved by the Invention

It is an objective of the present invention is to provide means for reducing the sulfur dioxide content in a processed composition in producing mannose and/or mannooligosaccharide from a raw material konjac flour (glucomannan) which is obtained by processing konjac potatoes.

Means for Solving the Problems

As a result of extensive efforts to find a way to achieve the objective, the present inventors have succeeded in finding that the sulfur dioxide content in the composition can be reduced by decomposing glucomannan derived from konjac potatoes, which are a raw material, followed by subjecting the resulting composition to heat treatment at a temperature of 105 °C or higher. As such, the present invention has been completed.

According to the present invention, the following aspects (1) to (6) are provided:

(1) A functional composition containing mannose and/or mannooligosaccharide derived from konjac flour, wherein the composition has a sulfur dioxide content of equal to or less than 0.03 g/kg dry weight.
(2) A method of producing the functional composition according to the item (1) characterized by subjecting a composition containing mannose and/or mannooligosaccharide derived from konjac flour to heat treatment at a temperature equal to or more than 105 °C.
(3) A food or beverage containing the functional composition according to the item (1).
(4) An enzymatic decomposition product of konjac flour containing mannose and/or mannooligosaccharide derived from konjac flour, wherein the product has a sulfur dioxide content of equal to or less than 0.03 g/kg dry weight.
(5) A method of producing the enzymatic decomposition product of konjac flour according to the item (4) characterized by subjecting glucomannan to enzymatic decomposition treatment followed by subjecting the resultant to heat treatment at a temperature equal to or more than 105 °C.
(6) A food or beverage containing the enzymatic decomposition product of konjac flour according to the item (4).

Effect of the Invention

According to the present invention, the sulfur dioxide content in the functional composition containing mannose and/or mannooligosaccharide can be easily reduced to 0.03 g/kg or less, thereby providing a food composition that fulfills the criteria under the Food Sanitation Law and is also safe.

Description of Embodiments

The present invention will now be described in detail below.
The konjac flour in the present invention may be in any form as long as the konjac flour is a processed product which is produced from konjac potatoes using flame drying. In general, the konjac flour is preferably a flour containing glucomannan, including rough powder, refined powder, and tobiko powder, which is a flour obtained by processing konjac potatoes. The konjac flour may be used as it is, or may be further refined to be used. Mixtures of the konjac flour may also be used. The further refined konjac flour may have been refined to improve the purity of glucomannan and is not particularly limited. Examples of the further refined konjac flour include konjac flour obtained by removing impurities using alcohol or hydrous alcohol, and konjac flour refined to remove odors by roasting or any other treatment.
Mannose is monosaccharide that is a C-2 epimer of glucose. Mannooligosaccharide is oligosaccharide that contains at least one or more molecules of mannose as a constituent saccharide. Mannooligosaccharide is composed of 2 to 20 molecules of monosaccharide including mannose as constitute saccharides with bound together, preferably 2 to 10 molecules of monosaccharide including mannose as constitute saccharides with bound together. Of course, mannooligosaccharide may include glucose in addition to mannose with bound together, and may include branched chains.
The mannose and/or mannooligosaccharide derived from konjac flour may be produced by any methods, as long as they are mannose and/or mannooligosaccharide produced using konjac flour as a raw material. The method of producing mannose and/or mannooligosaccharide by decomposing glucomannan contained in konjac flour is not particularly limited, but includes preferably methods including decomposing glucomannan using acids or enzymes. The acid to be used for acid hydrolysis is not particularly limited, but the examples of the acid include hydrochloric acid, sulfuric acid, acetic acid, formic acid, oxalic acid, phosphoric acid, fumaric acid and citric acid. The acid hydrolysis may be conducted under any conditions including conditions at a reaction temperature between 80 °C and 200 °C for 10 minutes to 24 hours. The enzyme to be used for the enzymatic decomposition is not particularly limited as long as the enzyme has an activity to release mannose and/or mannooligosaccharide from glucomannan. Examples of the enzyme include preferably mannanase, galactomannanase, glucomannanase, mannosidase, alpha-xylosidase, xyloglucanase, arabinanase, beta-xylosidase, xylanase, alpha-arabinofuranosidase and cellulase. Among them, the enzyme is preferably mannan-decomposing enzymes such as mannanase, glucomannanase, mannosidase and galactomannanase. Examples of commercially available mannan-decomposing enzyme include mannanase BGM "Amano" 10 (Amano Enzyme Inc.), Cellulosin GM5 (HBI Enzymes Inc.), Sumiteam ACH (Shin Nippon Chemical Industry Co., Ltd.), Sumiteam AC (Shin Nippon Chemical Industry Co., Ltd.) and Cellulosin TP25 (HBI Enzymes Inc.).

The enzyme reaction is preferably conducted under conditions without the enzyme deactivated, and examples of the enzyme reaction temperature include 30 °C to 75 °C, preferably 45 °C to 70 °C, and more preferably 50 °C to 65 °C. The pH range during the enzyme reaction may be selected according to the optimum pH range for the enzyme used. Examples of the pH range include preferably pH 2 to 9, more preferably pH 2.5 to 8, and even more preferably pH 3 to 6. While the enzyme reaction time depends on the amount of the enzyme used, the time may be appropriately selected for the optimal condition of, for example, from 1 hour to 3 days.

The content of sulfur dioxide (sulfurous acid, sulfite salt) in the functional composition according to the present invention is equal to or less than 0.03 g/kg in terms of dry weight. When the functional composition is in liquid form and the like, the weight of the composition is calculated per dry weight from wet weight taking into consideration the water content. The composition has a sulfur dioxide content of equal to or more than 0.03 g/kg is undesirable because the composition cannot be distributed as foods under the criteria of the Food Sanitation Law. In order to reduce the sulfur dioxide content to 0.03 g/kg or less, it is possible to reduce the sulfur dioxide content by heating the composition at a temperature of 105 °C or higher, preferably between 110 °C and 150 °C, and more preferably between 120 °C and 140 °C after completing the glucomannan decomposition treatment. The heating time may be from 5 minutes to 24 hours, preferably from 10 minutes to 2 hours, and more preferably from 10 minutes to 30 minutes.

The functional composition according to the present invention may be purified as appropriate after completing the reaction in order to further improve the contained ratio of mannose and/or mannooligosaccharide. While examples of the purification method include conducting decolorization with bone charcoal, activated charcoal, carbonic acid saturation, adsorption resins and magnesia preparation followed by desalination and deacidification with ion exchange resins, ion exchange membranes and electrodialysis, the purification method may apply any known methods. The combination of the purification method and the condition for purification may be selected as appropriate according to amounts of color matters, salts and acids in the reaction solution or other factors.

While the form of the functional composition according to the present invention is not particularly limited, examples of the form includes any form containing mannose and/or mannooligosaccharide such as solutions, dried powder or granules obtained by crystallization, spray-drying, freeze-drying or other drying and tablets shaped by tableting the composition in powder or granule form.

When the functional composition according to the present invention is added to foods and beverages, and oral administration pharmaceuticals, the foods and beverages may be easy to consume, and the oral administration pharmaceuticals may be easy to take, for example.

The above foods and beverages are not particularly limited, and examples of them include fruit drink or vegetable juices including citrus juices; carbonated drinks such as cola, ginger ale and cider; soft drinks such as sports drinks; tea drinks such as coffee, black tea and matcha green tea; general drinks including milk beverages such as cocoa and lactic acid bacteria beverages; desserts such as yogurt, jelly, pudding and mousse; confectioneries such as baked and steamed confectioneries including Western and Japanese sweets, for example, cakes and buns; sauces including fruit flavored sauces and chocolate sauces; sweets including chewing gum, hard candy, nougat candy and jelly beans.

While the dosage form of the above-mentioned oral administration pharmaceuticals is not limited, examples of the dosage form include solid dosage forms such as tablets, granules, and capsules, as well as liquid dosage forms such as solutions and suspensions.

Examples

The following examples are provided to further illustrate the method according to the present invention in detail, but the technical scope of the present invention is not limited to the following examples.

<Analysis of mannose and β-1,4-mannobiose>

    (1) Standard materials
Mannose (product name "D-Mannose research grade" manufactured by SERVA), β-1,4-mannobiose (manufactured by Megazyme).
    (2) Analytical methods (one of the following two methods was selected as appropriate)

(A) High performance liquid chromatography 1

Analytical column: Aminex HPX-87H (manufactured by Bio-Rad)
Column temperature: 50 °C; mobile phase: 0.005 M sulfuric acid; flow rate: 0.6 mL/min; detection: differential refractometer.

(B) High performance liquid chromatography 2

Analytical column: Aminex HPX-87P (manufactured by Bio-Rad)
Column temperature: 50 °C; mobile phase: water; flow rate: 0.6 mL/min; detection: differential refractometer.

<Analysis of sulfur dioxide>

Sulfur dioxide was analyzed by alkaline titration using a sulfite quantification apparatus AR-10 (Miyamoto Riken Ind. Co,. Ltd.). To a 50 mL conical tube, 10 mL of 0.3% hydrogen peroxide solution was added and three drops of methyl red-methylene blue test solution were added. Then, after adding 0.01 mL of 0.01 mol/L sodium hydroxide solution, the tube was attached to the apparatus. Each sample was weighed precisely and added to a 100 mL round-bottom flask. After adding 20 mL of milli-Q water, 2 mL of ethanol, two drops of silicone resin, and 10 mL of 25% phosphoric acid, the flask was attached to the apparatus. While ventilating nitrogen gas at a rate of 0.55 L/min, the round-bottom flask was heated for 10 minutes with the flame height of a micro gas burner set at 5 cm. The conical tube was then removed, and the solution was set as a test solution. Titration with 0.01 mol/L sodium hydroxide solution was carried out using a variable repetitive syringe dispenser 8100 (manufactured by NICHIRYO CO., LTD.), in which the endpoint of titration was reached when the solution turned green. The content of sulfur dioxide (g/kg) in the sample was calculated using the following formula:

Sulfur dioxide content (g/kg) = (titration volume of test solution (mL) - titration volume of blank solution (mL)) x factor of 0.01 mol/L sodium hydroxide solution x 0.32 x (1 / amount of sample collected (g)).

Example 1

Mannanase BGM "Amano" 10 (Amano Enzyme Inc.) was dissolved in water at a concentration of 0.01% by mass. To 1 g of konjac flour (manufactured by YUKIGUNI AGURI CO.,LTD.), 0.25 mL of the resulting enzyme solution and 10.25 mL of water were added, and the mixture was subjected to enzyme treatment at 58 °C for 48 hours and then subjected to heat treatment at 105 °C for 30 minutes. The reaction product was rotary evaporated to distill off all water. In the obtained viscous functional composition, the mannose concentration was 378.12 g/kg, and the sulfur dioxide concentration was 0.01 g/kg.

Example 2

Cellulosin GM5 (HBI Enzymes Inc.) was dissolved in water at a concentration of 0.01% by mass. To 1 g of konjac flour (manufactured by YUKIGUNI AGURI CO.,LTD.), 0.25 mL of the resulting enzyme solution and 10.25 mL of water were added, and the mixture was subjected to enzyme treatment at 58 °C for 48 hours and then subjected to heat treatment at 121 °C for 15 minutes. The reaction product was rotary evaporated to distill off all water. In the obtained viscous functional composition, the mannose concentration was 407.73 g/kg, and the sulfur dioxide concentration was 0.001 g/kg.

Example 3

Sumiteam ACH (Shin Nippon Chemical Industry Co., Ltd.) was dissolved in water at a concentration of 0.05% by mass. To 1 g of konjac flour (manufactured by YUKIGUNI AGURI CO.,LTD.), 0.25 mL of the resulting enzyme solution and 10.25 mL of water were added, and the mixture was subjected to enzyme treatment at 60 °C for 48 hours and then subjected to heat treatment at 121 °C for 20 minutes. The reaction product was rotary evaporated to distill off all water. In the obtained viscous functional composition, the $\beta$-1,4-mannobiose concentration was 150.67 g/kg, and the sulfur dioxide concentration was 0.002 g/kg.

Example 4

To 1 g of konjac flour (manufactured by YUKIGUNI AGURI CO.,LTD.), 10.5 mL of sulfuric acid (special grade chemical, FUJIFILM Wako Pure Chemicals Corporation) was added so that the sulfuric acid concentration became 60% by volume. The mixture was subjected to hydrolyzation treatment at 100 °C for 2 hours. Then, the resultant was neutralized with sodium hydroxide aqueous solution (special grade chemical, FUJIFILM Wako Pure Chemicals Corporation). Then, the resultant was subjected to heat treatment at 121 °C for 10 minutes. The reaction product was lyophilized to remove all water. In the obtained white functional composition, the mannose concentration was 130.45 g/kg, the $\beta$-1,4-mannobiose concentration was 34.89 g/kg, and the sulfur dioxide concentration was 0.001 g/kg.

Comparative Example 1

Mannanase BGM "Amano" 10 (Amano Enzyme Inc.) was dissolved in water at a concentration of 0.01% by mass. To 1 g of konjac flour (manufactured by YUKIGUNI AGURI CO.,LTD.), 0.25 mL of the resulting enzyme solution and 10.25 mL of water were added, and the mixture was subjected to enzyme treatment at 58 °C for 48 hours followed by heat treatment at 100 °C for 30 minutes. The reaction product was rotary evaporated to distill off all water. In the obtained viscous composition, the mannose concentration was 368.72 g/kg, and the sulfur dioxide concentration was 0.17 g/kg.

Comparative Example 2

Cellulosin GM5 (HBI Enzymes Inc.) was dissolved in water at a concentration of 0.01% by mass. To 1 g of konjac flour (manufactured by YUKIGUNI AGURI CO.,LTD.), 0.25 mL of the resulting enzyme solution and 10.25 mL of water were added, and the mixture was subjected to enzyme treatment at 58 °C for 48 hours followed by heat treatment at 100 °C for 15 minutes. The reaction product was rotary evaporated to distill off all water. In the obtained viscous composition, the mannose concentration was 409.63 g/kg, and the sulfur dioxide concentration was 0.35 g/kg.

Comparative Example 3

Sumiteam ACH (Shin Nippon Chemical Industry Co., Ltd.) was dissolved in water at a concentration of 0.05% by mass. To 1 g of konjac flour (manufactured by YUKIGUNI AGURI CO.,LTD.), 0.25 mL of the resulting enzyme solution and 10.25 mL of water were added, and the mixture was subjected to enzyme treatment at 60 °C for 48 hours followed by heat treatment at 100 °C for 20 minutes. The reaction product was rotary evaporated to distill off all water. In the obtained viscous composition, the $\beta$-1,4-mannobiose concentration was 160.13 g/kg, and the sulfur dioxide concentration was 0.15 g/kg.

Comparative Example 4

To 1 g of konjac flour (manufactured by YUKIGUNI AGURI CO.,LTD.), 10.5 mL of sulfuric acid (special grade chemical, FUJIFILM Wako Pure Chemicals Corporation) was added so that the sulfuric acid concentration became 60% by volume, and the mixture was subjected to hydrolyzation treatment at 100 °C for 2 hours. Then, the resultant was neutralized with sodium hydroxide (special grade chemical, FUJIFILM Wako Pure Chemicals Corporation). Then, the resultant was subjected to heat treatment at 100 °C for 10 minutes. The reaction product was lyophilized to remove all water. In the obtained white composition, the mannose concentration was 135.28 g/kg, the $\beta$-1,4-mannobiose concentration was 38.93 g/kg, and the sulfur dioxide concentration was 0.09 g/kg.

As is clear from the above examples, it was achieved to reduce the sulfur dioxide concentration in the functional composition to equal to or less than 0.03 g/kg by subjecting the composition to heat treatment at a temperature of 105 °C or higher. In contrast, the heat treatment at 100 °C employed in the comparative examples failed to reduce the concentration to equal to or less than 0.03 g/kg, rather was a level that would be regarded as a problem under the criteria of the Food Sanitation Law.

Cross-reference of related applications

The present application claims the benefit of priorities to Japanese Patent Application No. 2024-128105 filed on August 2, 2024, the disclosure of which is incorporated herein by reference in its entirety. The disclosure of all the documents described herein including Non-Patent Documents 1 to 3 and Patent Documents 1 to 6 is incorporated herein by reference in its entirety.

**Claims**

1.  A composition comprising mannose and/or mannooligosaccharide, wherein the mannose and the mannooligosaccharide is derived from konjac flour, wherein the composition has a sulfur dioxide content of equal to or less than 0.03 g/kg dry weight.

2. A method of producing the composition according to claim 1 **characterized by** subjecting an educt composition to heat treatment at a temperature equal to or more than 105 °C, wherein the educt composition comprises mannose and/or mannooligosaccharide, wherein the mannose and the mannooligosaccharide is derived from konjac flour.

3. The method according to claim 2, wherein the heat treatment is performed at a temperature of equal to or more than 110 °C.

4. The method according to claim 2, wherein the heat treatment is performed at a temperature of equal to or more than 115 °C.

5. The method according to any one of the preceding claims 2 to 4, wherein the heat treatment is performed at a temperature of equal to or less than 150 °C.

6. The method according to any one of the preceding claims 2 to 4, wherein the heat treatment is performed at a temperature of equal to or less than 140 °C.

7. The method according to any one of the preceding claims 2 to 6, wherein the heating time is from 5 minutes to 24 hours.

8. The method according to any one of the preceding claims 2 to 6, wherein the heating time is from 10 minutes to 2 hours.

9. The method according to any one of the preceding claims 2 to 6, wherein the heating time is from 10 minutes to 30 minutes.

10. A food or beverage comprising the composition according to claim 1.

Europäisches
Patentamt

European
Patent Office

Office européen
des brevets

## EUROPEAN SEARCH REPORT

**Application Number**

EP 25 19 3399

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 795 701 A1 (FUTAMURA KAGAKU KK [JP]; TOKYO INST TECH [JP]) 24 March 2021 (2021-03-24) * claims 1, 5, 11 * | 1-10 | INV. A23L29/244 A23L33/21 A23L5/20 |
| X | JP 2011 132187 A (SUNTORY HOLDINGS LTD) 7 July 2011 (2011-07-07) * claims 1, 8 * | 1-10 | |
| A | JP 2016 067290 A (DAICEL CORP) 9 May 2016 (2016-05-09) * the whole document * | 1-10 | |
| A | JP 2012 171899 A (UNITIKA LTD) 10 September 2012 (2012-09-10) * the whole document * | 1-10 | |

**TECHNICAL FIELDS SEARCHED (IPC)**

A23L

The present search report has been drawn up for all claims

| Place of search | Date of completion of the search | Examiner |
|---|---|---|
| The Hague | 23 November 2025 | Picout, David |

EPO FORM 1503 03.82 (P04C01)

EP 4 686 418 A1

**EP 4 686 418 A1**

## ANNEX TO THE EUROPEAN SEARCH REPORT
## ON EUROPEAN PATENT APPLICATION NO.

EP 25 19 3399

This annex lists the patent family members relating to the patent documents cited in the above-mentioned European search report.
The members are as contained in the European Patent Office EDP file on
The European Patent Office is in no way liable for these particulars which are merely given for the purpose of information.

23-11-2025

| Patent document cited in search report | Publication date | Patent family member(s) | Publication date |
|---|---|---|---|
| EP 3795701 A1 | 24-03-2021 | EP 3795701 A1<br>WO 2019220937 A1 | 24-03-2021<br>21-11-2019 |
| JP 2011132187 A | 07-07-2011 | JP 5960381 B2<br>JP 2011132187 A | 02-08-2016<br>07-07-2011 |
| JP 2016067290 A | 09-05-2016 | JP 6368609 B2<br>JP 2016067290 A | 01-08-2018<br>09-05-2016 |
| JP 2012171899 A | 10-09-2012 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011219469 A
- JP 4488852 B
- JP 5960381 B
- JP 2011254753 A
- JP 2019199462 A
- JP 2004254646 A
- JP 2024128105 A

**Non-patent literature cited in the description**

- Japan Society for Food Hygiene. Standards and Criteria for Foods and Food Additives (excerpt)
- **P. S. GONZALEZ et al.** Mannose impairs tumour growth and enhances chemotherapy.. *NATURE*, 2018, vol. 563, 719-723